# EUROPEAN PATENT SPECIFICATION

(11) **EP 4 027 931 B1**
(45) Date of publication and mention of the grant of the patent: **31.12.2025**
(21) Application number: 20774916.9
(22) Date of filing: 14.09.2020
(51) Int. Cl.: A61B 34/20, A61B 90/00

(54) **NAVIGATION UNIT AND METHOD**
NAVIGATIONSEINHEIT UND -VERFAHREN
UNITÉ DE NAVIGATION ET PROCÉDÉ

(30) Priority: 13.09.2019 US 201962899840 P
(43) Date of publication of application: 20.07.2022
(73) Proprietor: Fiagon GmbH, 16761 Hennigsdorf (DE)
(72) Inventor: DESINGER, Kai, 10711 Berlin (DE); MUCHA, Dirk, 16548 Glienicke (DE); NORMAN, Nicholas, CHARLOTTE, North Carolina 28203 (US)
(74) Representative: Eisenführ Speiser
(86) International application number: PCT/EP2020/075650
(87) International publication number: WO 2021/048439

(56) References cited:
- EP-A1- 3 506 278
- WO-A1-2007/017642
- WO-A1-2015/085011
- WO-A1-2017/100180
- WO-A2-2006/122001
- US-A1- 2014 200 621
- US-A1- 2016 213 430
- BRAINLAB AG: "CURVE DUAL NAVIGATION STATION - Technical User Guide Revision 1.1", 31 December 2017 (2017-12-31), XP055754456, Retrieved from the Internet <URL:https://userguides.brainlab.com/wp-content/uploads/2019/12/Curve-1.2-Technical-User-Guide-English-60915-69EN-Rev.1.1.pdf> [retrieved on 20201126]

## Description

### TECHNICAL FIELD

The invention relates to a navigation unit and a method for assisting a surgeon in navigating a medical instrument equipped with a localizer and a navigation system comprising such a navigation unit.

### BACKGROUND OF THE INVENTION

For assisting a surgeon in using a medical instrument in a surgical procedure it is known to track the position of the medical instrument inside a patient's body and to display the instrument's position in, e.g., sectional images of a model of a patient on a monitor.

To this end, navigation systems are used typically comprising a navigation unit, a monitor, and a number of localizers. A navigation unit often comprises a position detection unit that can be, e.g., an optical, an ultrasound-based or an electromagnetic position detection unit. A position detection unit, in general, is configured for determining position and orientation of localizers. The localizers can be mounted on a medical instrument to allows tracking of the medical instrument by means of the position detection system.

By way of example, electromagnetic position detection unit are known having a field generator for generating an alternating electromagnetic field. A medical instrument to be used with an electromagnetic position detection unit is equipped with a localizer that typically comprises one or more sensor coils.

When exposed to an alternating electromagnetic field, in the sensor coils of a localizer a voltage is induced that depends on the position and orientation of a respective sensor coil in the alternating electromagnetic field. By analysing a tapped voltage signal representing the induced voltage, position and orientation of the localizer can be determined. Typically, position and orientation of a localizer of a medical instrument are determined relative to the position and orientation of a reference localizer, sometimes called patient localizer, that can likewise comprise sensor coils and that stays fixed relative to a patient.

To be able to display the instrument's position in sectional images of a patient's model on a monitor of a navigation system, initially, the patient model has to be registered to the patient. Typically, a model of a patient is a topographic image that is generated from two-, three- or four-dimensional images of a patient obtained preoperatively by tomography, e.g., via computed tomography (CT), magnetic resonance imaging (MRI) or C-arm fluoroscopic imaging. Registration refers to obtaining the spatial correlation between position and orientation of a patient in real space (sometimes also called patient space) and the model, initially defined in terms of coordinates in the coordinate system of the respective two-, three- or four-dimensional image used for generating the model.

After having obtained the spatial relation between patient model and patient, e.g., by applying surface-based registration and point-based registration, the position of a medical instrument can be displayed in sectional images of the model for assisting a surgeon in navigating the medical instrument.

Various navigation systems are disclosed in WO 2015/085011 A1, US 2016/213430 A1, WO 2007/017642 A1, WO 2017/100180 A1, EP 3 506 278 A1, WO 2006/122001 A2, US 2014/200621 A1 and in Brainlab AG: "CURVE DUAL NAVIGATION STATION - Technical User Guide Revision 1.1".

### SUMMARY OF THE INVENTION

The invention is defined by the appended claims.

It is an object to provide an improved navigation unit, an improved navigation system comprising a navigation unit, and an improved method for assisting a surgeon in navigating a medical instrument equipped with a localizer.

With regard to the navigation unit the object is achieved by a navigation unit that is configured for assisting a surgeon in navigating a medical instrument equipped with a localizer.

The navigation unit comprises a housing, at least one localizer input port, a position detection unit, a state determination unit, a control unit and at least one operating state indicating light element.

The at least one localizer input port is configured to receive a localizer signal in case a localizer is operatively connected to the localizer input port.

A localizer to be connected to the localizer input port can be configured for capturing an electromagnetic navigation field (when exposed to such navigation field) and for providing a localizer signal accordingly.

The at least one operating state indicating light element is connected to the control unit and integrated in one of the housing walls such that light emitted from the operating state indicating light element is visible from outside the housing.

The navigation unit's housing accommodates at least the position detection unit, the state determination unit and the control unit.

The position detection unit is connected to the localizer input port for receiving a localizer signal and configured for processing the received localizer signal in order to determine position and orientation of the localizer, e.g., in the navigation field.

The state determination unit is connected to the position detection unit and configured for determining at least a localizer operating state of a connected localizer based on whether or not the position detection unit has received a localizer signal and whether or not the position detection unit has determined position and orientation of the localizer, e.g., in the navigation field, from a received localizer signal.

The control unit is connected to the state determination unit and configured for controlling the at least one operating state indicating light element at least based on the determined localizer operating state determined by the state determination unit.

The at least one operating state indicating light element is configured at least for visually indicating one of a plurality of localizer operating states. The at least one operating state indicating light element can be controlled by the control unit to visually indicate that localizer operating state of the localizer that has been determined by the state determination unit.

The invention includes the recognition that a surgeon using a navigation system for assistance in navigating a medical instrument commonly has to rely on that the position of the medical instrument displayed in sectional images of the patient on a monitor corresponds to the actual position of the medical instrument inside a patient's body. In particular, a surgeon has to rely on that the instrument's position is displayed correctly on the monitor throughout the full time of a navigated procedure. Furthermore, prior to starting a navigated procedure that is supported by a navigation system, the surgeon has to ascertain that the navigation system has booted up and is fully functional. Also prior to starting a navigated procedure, a surgeon has to ascertain that localizers to be used in the navigated procedure are fully functional. Moreover, the localizers have to be located at a distance to, e.g., a field generator of a navigation system, such that position and orientation of the localizes can be reliably determined with a navigation unit's position detection unit. Thus, for a surgeon it is beneficial, if the surgeon can quickly check whether a localizer is functioning as intended also during a navigated procedure.

Commonly, during a navigated procedure, a surgeon typically receives information on the functionality of a navigation system visualized, e.g., as text or symbols, on the navigation system's monitor. Thus, for delivering such information to a surgeon it is required that the surgeon looks at the monitor and catches the provided information. This sometimes includes that the information provided has to be perceived and processed by a surgeon next to a plurality of further information that are displayed on a monitor. In particular, if a surgeon is about to perform a difficult task of a navigated procedure, the surgeon often needs to strongly concentrate on the displayed position of a tracked medical instrument on the monitor. If in such a situation further information is provided to the surgeon via the monitor, the further information displayed on the monitor may interfere with the currently displayed information and distract a surgeon in a critical situation during a navigated procedure.

With the navigation unit according to the invention it is possible to provide information at least on the localizer operating state of a localizer that is connected to the navigation unit independently of a navigation system's monitor. In particular, the navigation unit itself is configured to visually indicate the present localizer operating state of a connected localizer. Advantageously, a surgeon using the navigation unit can obtain information on the localizer operating state of a connected localizer visually in a readily perceivable manner directly from the navigation unit at any time without interference with any information provided via a monitor.

In particular, with the navigation unit according to the invention the localizer operating state can be visually indicated in an easily perceivable way throughout a navigated procedure without distracting a surgeon while concentrating on information presented on a monitor. A surgeon can easily perceive the current localizer operating state simply by looking at the navigation unit.

The navigation unit according to the invention thus provides a device on its own for indicating at least the localizer operating state in a readily perceivable manner. The navigation unit can be part of a navigation system and, e.g., connected to a monitor for displaying the position of a medical instrument equipped with a localizer in sectional images of a patient on the monitor.

The aforementioned advantages of the navigation unit according to the invention can be achieved since with the navigation unit's state determination unit it is possible to determine the localizer operating state of a connected localizer and with the control unit it is possible to control the at least one operating state indicating light element to visually indicate the localizer operating state determined by the state determination unit. Hence, the determined operating state of a connected localizer can be indicated visually by the navigation unit itself in a readily perceivable manner and independently of information provided via a monitor of a navigation system.

To determine the localizer operating state of a connected localizer, the state determination unit is connected to the navigation unit's position detection unit and configured to obtain information from the position detection unit on whether the position detection unit has a received a localizer signal, thus, indicating whether or not a localizer is connected to the navigation unit's localizer input port. Furthermore, the state determination unit is configured to obtain information on whether the position detection unit has determined position and orientation of the localizer, e.g., in a navigation field, from a received localizer signal. The information on whether or not the position detection unit has received a localizer signal and whether or not the position detection unit has determined position and orientation of the localizer ,e.g., in the navigation field, from a received localizer signal can be actively pushed by the position detection unit to the state determination unit, e.g., each time a localizer signal is received by the position detection unit that has a signal-to-noise ratio that is above or below a predefined threshold. The information on whether or not the position detection unit has received a localizer signal and whether or not the position detection unit has determined position and orientation of the localizer, e.g., in the navigation field, from a received localizer signal can also be retrieved by the state determination unit, e.g., repeatedly in predefined time intervals.

For tracking the position and orientation of a localizer, typically, the localizer is exposed to a navigation field. Typically, a navigation field is generated by a field source, e.g., an electromagnetic field generator that generates an alternating electromagnetic field. The field strength of the generated electromagnetic field decreases with increasing distance from the field generator. Therefore, typically, a working space is defined in which position and orientation of a localizer can be determined in a reliable manner. For example, a working space can be defined in that within the working space, a localizer provides a localizer signal having a signal-to-noise ratio above a predefined threshold. In case a working space is defined, the state determination unit can also be configured to determine whether or not a localizer is within the working space based on the position and orientation of the localizer determined by the position detection unit.

For controlling the at least one operating state indicating light element based on the determined localizer operating state, the control unit is configured to use the determined localizer operating state. A signal representing the determined localizer operating state can, e.g., be actively pushed by the state determination unit to the control unit or the control unit can retrieve the most recently determined localizer operating state in predefined time intervals.

The control unit is configured to control the at least one operating state indicating light element based on the localizer operating state that has been determined by the state determination unit. In particular, the control unit is configured to control the at least one localizer state indicting light element to visually indicate that localizer operating state that has been determined by the state determination unit. For example, an operating state indicating light element that is assigned to a localizer input port used for connecting a localizer to the navigation unit can be switched on or off by the control unit to indicate the determined localizer operating state. It is also possible that an operating state indicating light element is controlled by the control unit to emit light at a different colour than before, wherein the new colour represents that localizer operating state that has been most recently determined by the state determination unit. It is also possible that the control unit controls the at least one operating state indicating light element such that the mode of emitting light by an operating state indicating light element is altered, e.g., from a continuous light emission mode to a pulsed light emission mode. Pulsed light emission can be realised by switching LEDs of the operating state indicating light element on and off at a specific frequency. In particular, different operating states can be visually indicated via a pulsed light emission in that a duty-cycle of a pulsed emission is adapted accordingly. A light emission mode that can be used for visually indicating a localizer operating state of a localizer can be a chase light effect simulating a movement of a light spot along a predefined path. A chase light effect can be implemented by controlling LEDs of a light element accordingly.

For visually indicating the determined localizer operating state, the navigation unit comprises at least one operating state indicating light element that is integrated in one of the housing walls such that light emitted from the operating state indicating light element is visible from outside the housing. In particular, the at least one operating state indicating light element is assigned to the at least one localizer input port and configured to visually indicate one of a plurality of localizer operating states. The at least one operating state indicating light element can be controlled by the control unit to visually indicate that localizer operating state that has been determined by the state determination unit.

Since the localizer operating state of a connected localizer can be indicated visually via the at least one operating state indicating light element that is assigned to the at least one input port, a surgeon can easily perceive the information by quickly looking at the navigation unit. Advantageously, the determined localizer operating state can be indicated by the at least one operating state indicating light element in a comparatively simple manner, e.g., by emitting light at a specific colour or by emitting light or not emitting light, i.e., switching the operating state indicating light element on or off.

Hence, when using the navigation unit according to the invention a user can easily register the current localizer operating state of a connected localizer that is visually indicated by respective operating state indicating light elements. Advantageously, a surgeon can check the determined localizer operating state quickly in a suitable situation without distracting the surgeon in conducting a navigated procedure. Advantageously, by visually indicating the localizer operating state, the information on the present localizer operating state is provided in a clear and readily perceivable manner. In particular, in case it is visually indicated to a surgeon that the localizer is within a working space, a surgeon can rely on that the position of a tracked medical instrument displayed on a monitor in sectional images of a patient comparatively accurately reflects the actual position of the medical instrument inside a patient's body. Likewise, if a surgeon moves a medical instrument equipped with the localizer outside a working space, the user gains feedback from the navigation unit that position and orientation of the localizer are determined less accurately.

In the following preferred embodiments of the navigation unit according to the invention are described.

Preferably, the at least one operating state indicating light element is assigned to the at least one localizer input port. In further preferred embodiments a plurality of operating state indicating light elements are provided, each of the operating state indicating light element being assigned to exactly one localizer input port in a one-to-one assignment.

The navigation unit can comprise several localizer input ports for operatively connecting several localizers at the same time to the navigation unit.

The navigation unit can comprise at least one input port for operatively connecting a medical instrument equipped with a localizer or for operatively connecting a medical instrument that does not comprise a localizer to the navigation unit. Preferably, a connected medical instrument is configured to provide an instrument signal to the navigation unit. Preferably, the state determination unit is configured for determining an instrument operating state based on a received instrument signal of a connected instrument. The control unit, preferably, is configured for controlling at least one operating state indicating light element based on the instrument operating state as determined by the state determination unit. An operating state indicating light element of the navigation unit, preferably, can be operated to visually indicate the determined instrument operating state.

It is possible that the same operating state indicating light element is used for subsequently visually indicating the operating state of different devices, e.g., first of a localizer and afterwards of an instrument. However, in general it is preferred that each operating state indicating light element is used for visually indicating the operating state of an associated device.

The state determination unit can be configured to determine the operating state of a workflow within software, in particular, of a workflow that is related to a navigated procedure. Workflows implemented by software that are related to a navigated procedure can comprise a localizer calibration workflow, a registration workflow, and/or a localizer navigation workflow. Based on the determined operating state of a workflow within software, the control unit can control at least one operating state indicating light element of the navigation unit such that the determined operating state of a respective workflow is visually indicated by the controlled operating state indicating light element.

An operating state that can be determined by the state determination unit can also be an operating state representing a localizer-medical instrument interaction. For example, such localizer-medical instrument interaction can be the calibration of an instrument tip to the position of a localizer that, e.g., can be held on the instrument or that can be a reference localizer used for calibration. An operating state representing a localizer-medical instrument interaction thus can be an operating state that is associated to a calibration process.

Preferably, via an input port of the navigation unit a tablet computer can be connected to the navigation unit. A tablet computer preferably comprises a touchscreen and software for controlling a data communication with the navigation unit for sending and receiving control signals and for generating control icons on the touchscreen. The tablet preferably is further configured to capture user inputs and to generate control signals for the Navigation unit accordingly. Preferably, a tablet computer can be used as a control interface device for controlling the navigation unit. By means of a camera of such a tablet computer it is possible to generate images used for registering a patient model to a patient prior to a navigated procedure. The state determination unit can be configured to determine whether or not a data communication to and from the tablet exists. The state determination unit can be further configured to determine an operating state of a tablet computer and based on the determined operating state of the tablet computer the control unit can control at least one operating state indicating light element to visually indicate the determined operating state of the tablet computer.

Preferably, a registration device, e.g., a photo registration camera, can be operatively connected to an input port of the navigation unit. A registration device is used for registering a patient model, e.g., a 2D or 3D model generated by means of tomography of a body part of a patient, to the patient being in an operating theatre, prior to a navigated procedure. The state determination unit can be configured to determine the operating state of a registration device, in particular, an operating state associated to a registration process. The control unit can be configured to control at least one operating state indicating light element to visually indicate that operating state of the registration device that has been determined by the state determination unit.

In a preferred embodiment, the at least one localizer input port is integrated in one of the housing walls to be accessible from outside the housing. The localizer input port can be configured for mechanically and electrically connecting a localizer to the navigation unit.

Alternatively or additionally, the localizer input port can be configured to be wirelessly connected to a localizer. In such embodiment, the localizer input port can be arranged within the space encapsulated by the walls of the housing.

Via the localizer input port, a localizer of a medical instrument can be operatively connected to the navigation unit. An operative connection between the localizer and the localizer input port is a connection that facilities a signal transmission between the localizer and the localizer input port. Such connection can be wireless or wirebound and may also include a mechanical connection. From a localizer signal provided by the localizer of the medical instrument, position and orientation of the instrument and, in particular, of the instrument's tip can be calculated. The calculated position and orientation of the instrument tip can be displayed in sectional images of a patient on a monitor of a navigation system.

Via the localizer input port, also a patient localizer can be operatively, e.g., mechanically and electrically, connected to the navigation unit. A patient localizer, typically, is arranged directly at a patient or at a fixed relative distance to the patient within a working space. The arranged patient localizer provides a localizer signal representing position and orientation of the patient localizer, e.g., in the navigation field.

Preferably, the navigation unit has at least two localizer input ports such that to one of the localizer input ports a localizer of a medical instrument can be connected and to the other localizer input port a patient localizer can be connected.

The state determination unit can be configured for determining whether the patient localizer is arranged at such a position within a working space at which position and orientation of the patient localizer can be reliably determined. The state determination unit can be configured for applying one or more reliability criteria for determining a position at which position and orientation of the patient localizer can be reliably determined.

Preferably, the state determination unit is configured to check whether or not the signal-to-noise ratio of a received patient localizer signal is above a predefined threshold value and if the signal-to-noise ratio is found to be above the predefined threshold value to provide that a patient localizer position is determined at which position and orientation of the patient localizer can be reliably determined. The control unit can be configured to control at least one operating state indicating light element to visually indicate that the patient localizer is located at a suitable position, e.g., by emitting green light if a suitable position is determined and else emitting red light.

For finding a position at which position and orientation of the patient localizer can be reliably determined, preferably, the patient localizer is moved to different positions that are determined with the position detection unit. The state determination unit can be configured to determine the signal-to noise ratio of a patient localizer signal provided at each of the positions and to compare the signal-to-noise ratios in order to find that position at which position and orientation of the patient localizer can be determined comparatively reliably. Alternatively or additionally, the state determination unit can be configured to determine a position at which position and orientation of the patient localizer can be reliably determined based on the determination of a distortion measurements of a navigation field at each of the positions. The state determination unit can be configured to determine a distortion of a navigation field at a position of a patient localizer by comparing the patient localizer position as determined by the position detection unit to the theoretical position of the patient localizer as calculated in a non-distorted theoretical navigation field. Preferably, the state determination unit is configured to check whether or not a deviation of a detected patient localizer position from the theoretical position of the patient localizer calculated in a non-distorted navigation field model is smaller than a predefined threshold value and if the determined deviation is smaller to provide that a patient localizer position is determined at which position and orientation of the patient localizer can be reliably determined.

Preferably, the control unit is configured to control at least one operating state indicating light element to visually indicate whether a patient localizer is arranged at a position at which position and orientation of the patient localizer can be determined in a comparatively reliable manner. For example, in case a suitable patient localizer position has been determined the operating state indicating light element can be controlled by the control unit to emit green light instead of red light.

Hence, a user trying to find a suitable patient localizer position can move the patient localizer throughout different positions in a working space until the signal light emitted by an operating state indicating light element turns green thus indicating that a suitable position for him to fix the patient localizer to a patient is found.

The state determination unit can also be configured for determining a position within a working space at which an instrument can be calibrated with a reference localizer in a comparatively reliable manner. For determining a suitable calibration position, the state determination unit can be configured for using a number of reference localizer positions determined by the position detection unit and, e.g., signal-to-noise ratios and/or distortion measurements associated to the respective positions. The control unit can be configured to control at least one operating state indicating light element to visually indicate whether a suitable calibration position is found. Thereby, a user can be guided to a suitable calibration position within a working space.

The navigation unit's position detection system can be can be an optical or an ultra-sound based or, as it is preferred, an electromagnetic position detection system. In particular, an electromagnetic position detection system typically comprises a field generator for generating an alternating electromagnetic field. The field generator can be accommodated in the housing of the navigation unit or can be an external device.

If the navigation unit's position detection system is an electromagnetic position detection system, a localizer that is connected to the navigation unit, preferably, comprises one or more sensor coils for capturing a generated electromagnetic field and for providing localizer signals representing respectively induced voltages. For example, a 6DOF (degrees of freedom) sensor can be implemented with a localizer comprising two orthogonally arranged sensor coils.

Advantageously, the navigation unit according to the invention can be used for tracking position and orientation of localizer and for calculating position and orientation of a medical instrument equipped with the localizer. The calculated position and orientation can be used for displaying the position of the medical instrument in sectional images of a patient model visualized on a monitor connected to the navigation unit for supporting a surgeon in navigating the medical instrument.

Localizer operating states can be specified by values, e.g., a predefined set of values, of selected signal-to-noise ratios of a localizer signal. The state determination unit can be configured to use a signal-to-noise ratio criterion to determine the localizer operating state of a connected localizer.

The at least one operating state indicating light element can comprise one or more LEDs.

One or more LEDs of an operating state indicating light element can be covered by a light scattering cover to distribute light emitted by a LED over an area that is larger than the area of the LED. A cover can have, e.g., a round or rectangular area and can have a diameter that is, e.g., between 0.5 cm and 5 cm. It is also possible to use a cover having the shape of a stripe covering a plurality of LEDs arranged in line. Such a stripe can also extend along several housing walls such that light emitted can be seen from several viewing directions.

In case the at least one operating state indicating light element comprises several LEDs, LEDs emitting light at the same colour, i.e., at the same wavelength, or LEDs emitting light at different colours can be used for visually indicating a determine localizer operating state.

If the at least one operating state indicating light element comprises several LEDs, the operating state indicating light element can be connected to the control unit and configured such that for visually indicating the determined localizer operating state each LED can be controlled individually, or groups of LEDs, e.g., of LEDs emitting light at the same colour, can be controlled individually, or all LEDs can be controlled together, e.g., to increase the total light intensity.

If a plurality of localizer input ports are integrated in a housing wall, preferably, for each individual localizer input port at least one dedicated operating state indicating light element is integrated in the housing wall and assigned to the respective localizer input port. In particular, for each localizer input port one dedicated operating state indicating light element is provided that can emit different light signals under the control of the state determination unit via the control unit in dependence of a respective localizer operating state as determined by the state determination unit.

In some embodiments, the localizer input port can be a port for connecting more than one individual localizer. In such embodiment, preferably for each localizer that optionally can be connected the localizer input port an individual, dedicated operating state indicating light element is provided. If, for instance, a splitter cable is connected to such localizer input port allowing for multiple instruments to be connected at once, then numbers or other symbols on or next to the respective operating state indicating light element allow the user to identify the correct operating state indicating light element for a particular localizer connected to that specific localizer input port. In case e.g. three localizers may be connected to the localizer input port but only one of them is active and exposed to the electromagnet navigation field, this can be indicated by the assigned operating state indicating light element so the user knows for instance that the localizer in port 1 of the splitter cable is the current active localizer in the electromagnetic navigation field.

The state determination unit can be configured to base the determination on a signal-to-noise ratio of a localizer signal provided by the connected localizer. The state determination unit can be configured to determine the localizer operating state of a connected localizer in that it assigns a signal-to-noise ratio value or range to the localizer operating state. For example, the state determination unit can assign signal-to-noise ratio values or ranges to different localizer operating states of a connected localizer for determining the present localizer operating state of the localizer.

The state determination unit can be configured to use a plurality of threshold values each assigned to a different localizer operating state for determining the localizer operating state of a connected localizer.

Alternatively or additionally, the state determination unit can be configured to analyse a signal pattern of a localizer signal provided by a connected localizer for determining its localizer operating state. In particular, by analysing a signal pattern of a localizer signal it is possible to determine whether a connected localizer is fully functional or defect since a signal pattern of a defect localizer, e.g., can include signal fragments representing signal noise such that the signal-to-noise ratio of a received signal within the analysed signal pattern may change repeatedly.

The state determination unit can comprise a memory for storing a number of operating states of at least one localizer. For example, for a localizer to be connected to the navigation unit a fixed number of predefined localizer operating states can be stored in the memory and used by the state determination unit for determining the localizer operating state of that localizer. In particular, if the state determination unit comprises a memory, the state determination unit can be configured for determining the localizer operating state of a connected localizer of which localizer operating states are stored in the memory by assigning a localizer signal received by the position detection unit to one of the stored localizer operating states of the connected localizer. The assignment can be conducted, e.g., using the value of the signal-to-noise ratio of a received localizer signal.

If the state determination unit comprises a memory for each localizer to be connected to the navigation unit, a number of individual predefined operating states can be stored in the memory of the state determination unit.

Preferably, the state determination unit is configured for determining whether a localizer is connected to the navigation unit, i.e., active, and/or a connected localizer is not functioning, i.e., inactive, or not functioning to full extend, and/or a connected localizer is within a working space in which a signal-to-noise ratio of a localizer signal provided by the localizer of the medical instrument is above a predefined threshold. In particular, these localizer operating states can be the localizer operating states of a localizer of a medical instrument. The state determination unit can also be configured for determining whether a localizer is at rest at the moment indicating, e.g., that a medical instrument is currently not being used.

The state determination unit can also be configured for determining whether a localizer used as a patient localizer is connected to the navigation unit, and/or a connected patient localizer is not functioning or not functioning to full extend, and/or a connected patient localizer is detecting a distortion or interference of the navigation field, and/or a connected patient localizer is within a working space in which a signal-to-noise ratio of a patient localizer signal provided by the patient localizer is above a predefined threshold. A patient localizer, typically, stays fixed relative to a patient and is used for determining position and orientation of localizer of a medical instrument relative to the patient localizer's position and orientation.

Preferably, for visually indicating the determined localizer operating state, the at least one operating state indicating light element assigned to the at least one localizer input port can be controlled to be switched on or off, can be controlled to emit light at a different colour, can be controlled to emit light at an increased intensity and/or can be controlled to change a light emission mode.

The navigation unit according to the invention can further comprise at least one internal state indicating light element. If the navigation unit comprises an internal state indicating light element, preferably, the state determination unit is configured for determining an internal operating state of the navigation unit, and the control unit, preferably, is configured for controlling the at least one internal state indicating light element based on the internal operating state of the navigation unit as determined by the state determination unit.

Preferably, the at least one internal state indicating light element is configured to visually indicate one of a plurality of internal operating states of the navigation unit. Preferably, the at least one internal state indicating light element can be controlled by the control unit to visually indicate that internal operating state that has been determined by the state determination unit. The at least one internal state indicating light element can be configured the same way as described before for the at least one operating state indicating light element. Thus, the at least one internal state indicating light element can comprise one or more LEDs that can be covered by a light scattering cover.

Preferably, the at least one internal state indicating light element can be operated in various operation modes, each operation mode being assigned to a different localizer operating state of the navigation unit, wherein the at least one internal state indicating light element is controlled by the control unit to operate in that operation mode that is assigned to the determined internal operating state.

Preferably, the plurality of internal state indicating light element operation modes comprise a switched on mode, one or more colour modes, one or more light intensity modes, a chase light effect mode, one or more pulsed light modes.

Preferably, the at least one operating state indicating light element can be operated in various operation modes, each operation mode being assigned to a different localizer operating state of a connected localizer. Preferably, the at least one operating state indicating light element is controlled by the control unit to operate in that operation mode that is assigned to the determined localizer operating state.

Preferably, the plurality of operating state indicating light element operation modes comprise a switched on mode, one or more colour modes, one or more light intensity modes, a chase light effect mode, one or more pulsed light modes. A pulsed light mode can be characterized by a specific duty-cycle or a more complex pulse rhythm.

Preferably, the state determination unit is configured for determining whether the navigation unit is booting up, and/or is in a calculating state, and/or is in a navigation mode, and/or requires user interaction, and/or whether the determination of position and orientation of a localizer connected to the navigation unit is inaccurate or interrupted, and/or if a localizer connected to the navigation unit leaves a working space, and/or if an error is detected.

A calculating state of the navigation unit can comprise calculating the registration of a patient model to a patient or calculating the calibration of an instrument tip of a medical instrument to the position of a localizer of the medical instrument. In a navigation mode, position and orientation of a localizer connected to the navigation unit can be determined, e.g., such that the position of a medical instrument can be displayed in sectional images of a patient on a monitor. In particular, the navigation unit can be defined to be in a navigation mode, if a localizer connected to the navigation unit is located within a working space. A detected error can be a detected registration error or a detected calibration error. If a registration error or a calibration error is detected and visually indicated to a surgeon, the surgeon knows that registration or calibration have to be repeated in order to use the navigation unit as intended. A warning message can be visually indicated, e.g., if a localizer is leaving the working space, and/or if field inhomogeneities of a navigation field are detected such that the determined position and orientation of a localizer may not be accurate. If a warning message is visually indicated to a user, the user is motivated, e.g., to look at a monitor for detailed instructions. If it is visually indicated by the navigation unit that user action is required, a user may provide input via a user interface.

The state determination unit and the control unit can be implemented, e.g., by means of a microcontroller and software. A microcontroller can comprise sensors for determining the localizer operating state of a connected localizer, e.g., based on the signal-to-noise ratio of a received localizer signal. A microcontroller can also comprise an ADC (analog-digital converter) and a number of switches that can be controlled for switching LEDs of, e.g., the at least one operating state indicating light element and/or the at least one internal state indicating light element on and off.

With regard to the navigation system the aforementioned object is achieved by a navigation system for assisting a surgeon in navigating a medical instrument equipped with a localizer. The navigation system comprises navigation unit, a monitor, and a localizer of a medical instrument and/or a patient localizer.

The navigation unit, preferably, is configured according one of the embodiments of the navigation unit according to the invention described herein. The localizer of a medical instrument is configured for capturing a navigation field and for providing a localizer signal. The localizer is mechanically and electrically connected to a first localizer input port of the navigation unit. Alternatively or additionally to the localizer of a medical instrument, the navigation system comprises patient localizer. The patient localizer is configured for capturing a navigation field and for providing a patient localizer signal. If present, the patient localizer can be operatively, e.g., mechanically and electrically, connected to a second localizer input port of the navigation unit.

The monitor is connected to the navigation unit and configured for displaying a position of the medical instrument.

Preferably, when using the navigation system for supporting a surgeon in a navigated procedure, the navigation unit is arranged such that during the navigated procedure the navigation unit is located within the field of view of the surgeon when performing surgery. For example, the navigation unit can be arranged next to the monitor or behind a patient.

With regard to the method the aforementioned object is achieved by a method for visually indicating a localizer operating state of a localizer. The method comprises the steps of
- operatively connecting a localizer to a navigation unit, the localizer being configured for capturing a navigation field and for providing a localizer signal,
- generating a navigation field for determining position and orientation of the localizer in the navigation field,
- receiving a localizer signal from the localizer and processing the received localizer signal in order to determine position and orientation of the localizer in the navigation field,
- determining a localizer operating state based on the received localizer signal and whether or not the received localizer signal represents position and orientation of the localizer in the navigation field, and
- controlling at least one operating state indicating light element that is assigned to a localizer input port used for connecting the localizer to the navigation unit to indicate visually the determined localizer operating state.

The method can further comprise the steps of
- determining an internal operating state of the navigation unit, and
- controlling at least one internal state indicating light element to indicate visually the determined internal operating state.

The invention also refers to a program means that is configured for
- determining a localizer operating state based on whether or not a position detection unit has received a localizer signal of a localizer and whether or not a position detection unit has determined position and orientation of a localizer in a navigation field from a received localizer signal and/or for determining an internal operating state of the navigation unit, and
- outputting the determined localizer operating state of a localizer and/or the determined internal operating state of the navigation unit.

The invention further refers to a persistent storage medium on which the program means as described before is stored.

### BRIEF DESCRIPTION OF THE DRAWINGS

In the following, preferred embodiments of the invention are described with reference to the figures. In the figures:
Fig. 1:schematically shows the components of a navigation unit having operating state indicating light elements and internal state indicating light;
Fig. 2:shows a photograph of a navigation unit having operating state indicating light elements and internal state indicating light elements integrated in housing walls;
Fig. 3:schematically shows a navigation system comprising a navigation unit;
Fig. 4:shows a flow diagram representing a method for visually indicating a localizer operating state of a localizer.

### DETAILED DESCRIPTION

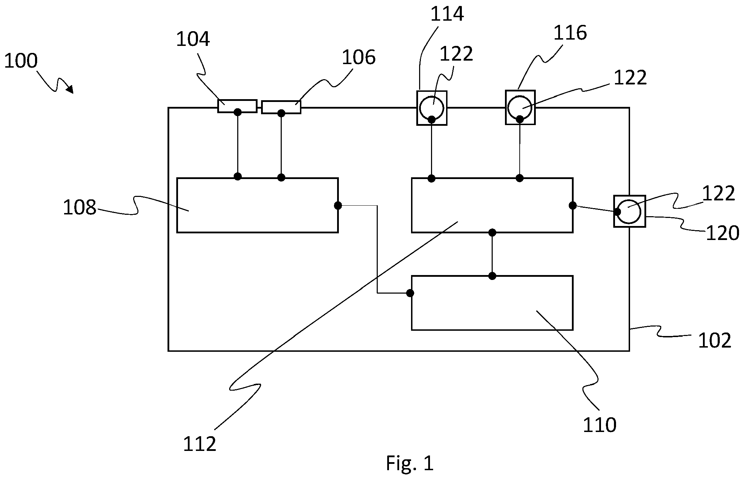

Figure 1 schematically shows the components of a navigation unit 100 having operating state indicating light elements and an internal state indicating light.

The navigation unit 100 comprises a housing 102 having housing walls. In the housing walls two localizer input ports 104, 106 are integrated. Localizer input ports 104, 106 are accessible from outside the housing 102 and configured such that to each of the localizer input ports 104, 106 localizers (not shown) can be mechanically and electrically connected, respectively. In particular, localizers can be connected to the localizer input ports 104, 106 that are configured for capturing a navigation field and for providing a localizer signal.

For example, localizers can comprise one or more sensor coils for capturing an alternating electromagnetic field generated by a field generator. When exposing the sensor coils to the alternating electromagnetic field, a voltage is induced in each of the coils and each of the coils provides a localizer signal representing the induced voltage.

The localizer input ports 104, 106 can be used for connecting a localizer of a medical instrument to the navigation unit 100. The second one of the localizer input ports 104, 106 can be used for connecting a patient localizer to the navigation unit 100. A patient localizer, typically, is fixedly arranged at a patient and the position of a localizer of a medical instrument can be tracked relative to the position of the patient localizer. It is also possible, to use localizer input ports 104, 106 for connecting two localizers of two respective medical instruments to the navigation unit 100. Position and orientation of each of the two medical instruments can be determined by the navigation unit 100 at the same time such that a user can use two tracked medical instruments simultaneously in a navigated procedure.

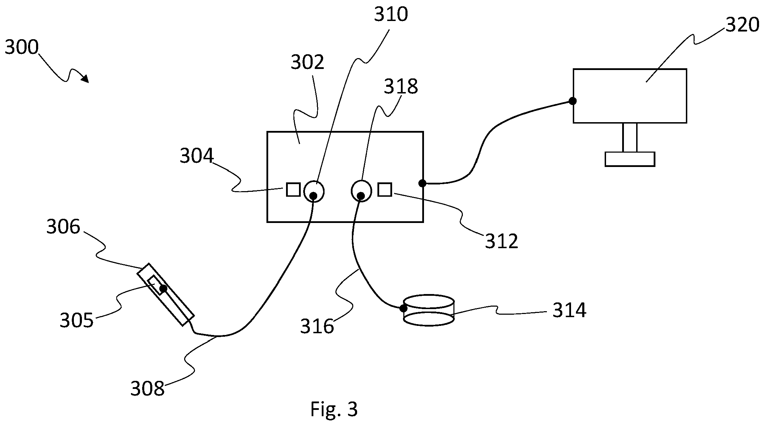

The localizer input ports 104, 106 are connected to a position detection unit 108. The position detection unit 108 is configured for receiving a localizer signal of a connected localizer and for processing the received signal in order to determine position and orientation of the localizer in a navigation field. The navigation unit 100 can be connected to a monitor (not shown) of a navigation system, e.g., a navigation system as described with respect to figure 3, in order to display the position of a medical instrument is equipped with the localizer in sectional images of a patient model obtained via tomography.

The position detection unit 108 is connected to a state determination unit 110. The state determination unit 110 is configured for determining a localizer operating state of a connected localizer based on whether or not the position detection unit 108 has received a localizer signal and whether or not the position detection unit 100 has determined position and orientation of a localizer in the navigation field from a received localizer signal. The state determination unit 110 is configured for determining whether a localizer is connected to the navigation unit 100 and whether or not the connected localizer is located within a working space. A working space can comprise those localizer positions at which a signal-to-noise ratio of a localizer signal provided by the localizer of a medical instrument is above a predefined threshold. For determining the localizer operating state of a connected localizer, the state determination unit 110 can be configured to use the signal-to-noise ratio of a received localizer signal. The state determination unit 110 can also be configured to analyse a signal pattern of a received localizer signal for determining the localizer operating state of the connected localizer.

The state determination unit 110 is further configured for determining and internal operating state of the navigation unit 100. An internal operating state of the navigation unit 100 can be that the navigation unit 100 is booting up, is in a calculating state, is in a navigation mode, requires user action, has detected an error, has determined that the connected localizer is leaving the working space, or has detected that the determination of position and orientation is inaccurate.

The state determination unit is connected to a control unit 112. The control unit 112 is connected to a first operating state indicating light element 114 that is used for visually indicating a localizer operating state of a first localizer, e.g., a patient localizer, connected, e.g., to localizer input port 104. The control unit 112 is also connected to a further operating state indicating light element 116 that is used for visually indicating a localizer operating state of a localizer, e.g., a localizer of a medical instrument, connected to localizer input port 106. The control unit 112 is also connected to an internal state indicating light element 120. The internal state indicating light element 120 is used for visually indicating an internal operating state of the navigation unit 100.

Each of the operating state indicating light elements 114, 116, can comprise one or more LEDs 122 and the internal state indicating light element 120, too, can comprise one or more LEDs 122. Each of the light elements 114, 116, 120 can comprise a light scattering cover that is covering the LEDs for scattering light emitted by the LEDs.

Each of the LEDs 122 of the light elements 114, 116, 120, preferably, can be controlled individually by the control unit 112. Individually controlling the LEDs 122 can comprise controlling switches assigned to each of the LEDs in order to switch on and off individual LEDs to visually indicate the determined localizer operating state of a connected localizer and/or the determined internal operating state of the navigation unit 100. For example, the light elements 114, 116, 120, can comprise several LEDs each emitting light at a different colour.

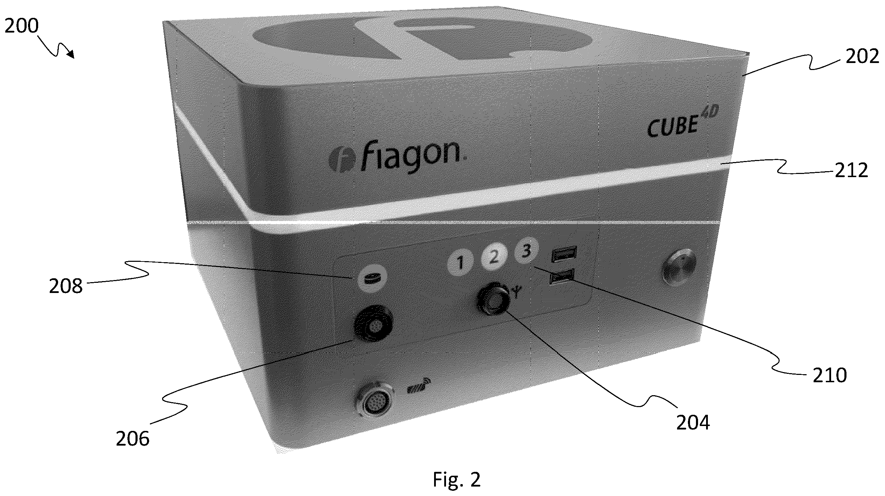

In figure 2, a navigation unit 200 is shown having a housing 202 in which a state determination unit, a position detection unit, and a control unit as described with reference to figure 1 can be accommodated.

In a housing wall of housing 202 a first localizer input port 204 for connecting a localizer of a medical instrument is integrated. In the same housing wall of housing 202 a second localizer input port 206 is integrated for connecting a patient localizer to the navigation unit 200. An operating state indicating light element 208 is integrated in the housing wall next to the localizer input 206 and assigned to the second localizer input port 206. The operating state indicating light element 208 comprises a light scattering cover showing schematically a patient localizer in order to provide that this operating state indicating light element 208 is used for visually indicating a localizer operating state of a connected patient localizer.

Next to the first localizer input port 204, three spatially separated operating state indicating light elements 210 are integrated in the housing wall. Each of the operating state indicating light elements 210 comprises a light scattering cover showing one of the numbers 1, 2, or 3 referring to three different localizer input ports. Each of the three operating state indicating light elements 210 is assigned to a different localizer input port and used to visually indicate the localizer operating state of a localizer connected to the respective localizer input port.

Integrated in the housing walls of housing 202 there is an internal state indicating light element 212 comprising a plurality of LEDs that are arranged in a line along the circumference of the navigation unit 200 such that an internal operating state of the navigation unit 200 visually indicated by the internal state indicating light element 212 can be visually perceived from various directions. The LEDs of internal state indicating light element 212 are covered by a light scattering cover in form of a stripe running along the circumference of the navigation unit 200. The internal state indicating light element can be operated in different operation modes for visually indicating the determined internal operating state of the navigation unit. Operation modes in which the at least one internal state indicating light element can be operated, preferably, comprises a switched on mode, one or more colour modes, one or more light intensity modes, a chase light effect mode, one or more pulsed light modes.

Figure 3 schematically shows a navigation system 300 comprising a navigation unit 302 that can be a navigation unit as described with reference to figures 1 or 2.

The navigation unit 302 has an operating state indicating light element 304 that is used to visually indicate the operating state of a localizer 305 of a medical instrument 306 that is connected via a cable 308 to a first localizer input port 310 integrated in a housing wall of navigation unit 302.

The navigation unit 302 has another operating state indicating light element 312 that is used for indicating visually a localizer operating state of a patient localizer 314 that is connected via a cable 316 to a second localizer input port 318 of the navigation unit 302. The patient localizer 314 can be used to provide a reference position relative to which the position of the localizer 305 of the medical instrument 306 can be determined.

The navigation unit 302 is connected to a monitor 320 for displaying the position of the medical instrument 306 in sectional images of a patient for assisting a surgeon in navigating the medical instrument 306 inside a patient's body.

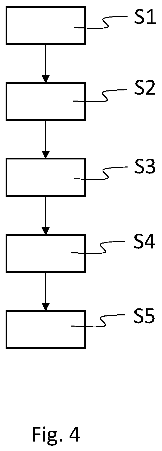

The navigation system 300 can be used for conducting a method for visually indicating a localizer operating state of a localizer as represented by the flow diagram described with reference to figure 4.

In a first step S1 of that method for visually indicating a localizer operating state of a localizer, a localizer is mechanically and electrically connected to a navigation unit. The localizer is configured for capturing a navigation field and for providing a localizer signal.

For determining position and orientation of the localizer in a navigation field, a navigation field is generated in step S2, e.g., by a field generator. For example, a field generator can be used for generating an alternating electromagnetic field. When exposing the localizer having sensor coils to the electromagnetic field, a voltage is induced in the coils that depends on position and orientation of the localizer in the electromagnetic field.

In step S3, a localizer signal provided by the localizer is received and the received localizer signal is processed in order to determine position and orientation of the localizer in the navigation field. Based on the received localizer signal and whether or not the received localizer signal represents position and orientation of the localizer in the navigation field, in step S4, a localizer operating state is determined.

In step S5, at least one operating state indicating light element that is assigned to a localizer input port used for connecting the localizer to the navigation unit is controlled to indicate visually the determined localizer operating state.

In case the navigation system 300 described with reference to figure 3 additionally comprises at least one internal state indicating light element, with the navigation system 300 also optional method steps of the method described with reference to figures 4 can be conducted, the optional method steps comprising determining an internal operating state of a navigation unit and controlling at least one internal state indicating light element to indicate visually the determined internal operating state.

## Claims

1. A navigation unit that is configured for assisting a surgeon in navigating a medical instrument (306) equipped with a localizer, the navigation unit comprising a housing (102, 202) with walls, at least one localizer input port, a position detection unit, a state determination unit (110), a control unit (112) and at least one operating state indicating light element, wherein
- the at least one localizer input port being configured for receiving a localizer signal provided by a localizer operatively connected to the localizer input port, and
- the at least one operating state indicating light element being connected to the control unit (112) and integrated in one of the housing (102, 202) walls such that light emitted from the operating state indicating light element is visible from outside the housing (102, 202),
the housing (102, 202) accommodating at least
- the position detection unit that is connected to the localizer input port for receiving a localizer signal and configured for processing the received localizer signal in order to determine position and orientation of the localizer,
- the state determination unit (110) that is connected to the position detection unit and configured for determining at least a localizer operating state of a connected localizer based on whether or not the position detection unit has received a localizer signal and whether or not the position detection unit has determined position and orientation of the localizer from a received localizer signal, and
- the control unit (112) that is connected to the state determination unit (110) and configured for controlling the at least one operating state indicating light element at least based on the localizer operating state determined by the state determination unit (110),
wherein the at least one operating state indicating light element is configured at least for visually indicating one of a plurality of localizer operating states and can be controlled by the control unit (112) at least to visually indicate that localizer operating state that has been determined by the state determination unit (110).

2. The navigation unit of claim 1, wherein the state determination unit (110) is configured to base the determination on a signal-to-noise ratio of a localizer signal provided by the connected localizer.

3. The navigation unit of claim 1 or 2, wherein the state determination unit (110) is configured to use a plurality of threshold values each assigned to a different localizer operating state for determining the localizer operating state of a connected localizer.

4. The navigation unit of at least one of claims 1 to 3, wherein the state determination unit (110) is configured to analyse a signal pattern of a localizer signal provided by a connected localizer for determining its localizer operating state.

5. The navigation unit of at least one of claims 1 to 4, wherein the state determination unit (110) comprises a memory for storing a number of operating states of at least one localizer and wherein the state determination unit (110) is configured for determining the localizer operating state of a connected localizer of which localizer operating states are stored in the memory by assigning a localizer signal received by the position detection unit to one of the stored localizer operating states of the connected localizer.

6. The navigation unit of at least one of claims 1 to 5, wherein the state determination unit (110) is configured for determining whether a localizer is connected to the navigation unit, and/or a connected localizer is not functioning or not functioning to full extend, and/or a connected localizer is within a working space in which a signal-to-noise ratio of a localizer signal provided by the localizer of the medical instrument (306) is above a predefined threshold.

7. The navigation unit of at least one of claims 1 to 6, wherein the state determination unit (110) is configured for determining whether a localizer used as a patient localizer (314) is connected to the navigation unit, and/or a connected patient localizer (314) is not functioning or not functioning to full extend, and/or a connected patient localizer (314) is detecting a distortion or interference of the navigation field, and/or a connected patient localizer (314) is within a working space in which a signal-to-noise ratio of a patient localizer signal provided by the patient localizer (314) is above a predefined threshold.

8. The navigation unit of at least one of claims 1 to 7, wherein the at least one operating state indicating light element can be operated in various operation modes, each operation mode being assigned to a different localizer operating state of a connected localizer, wherein the at least one operating state indicating light element is controlled by the control unit (112) to operate in that operation mode that is assigned to the determined localizer operating state.

9. The navigation unit of at least one of claims 1 to 8, further comprising at least one internal state indicating light element, wherein
- the state determination unit (110) is configured for determining an internal operating state of the navigation unit,
- the control unit (112) is configured for controlling the at least one internal state indicating light element based on the internal operating state of the navigation unit determined by the state determination unit (110),
wherein the at least one internal state indicating light element is configured to visually indicate one of a plurality of internal operating states of the navigation unit and can be controlled by the control unit (112) to visually indicate that internal operating state that has been determined by the state determination unit (110).

10. The navigation unit of claim 9, wherein the state determination unit (110) is configured for determining whether the navigation unit is booting up, and/or is in a calculating state, and/or is in a navigation mode, and/or requires user interaction, and/or whether the determination of position and orientation of a localizer connected to the navigation unit is inaccurate or interrupted, and/or if a localizer connected to the navigation unit leaves a working space, and/or if an error is detected.

11. A navigation system (300) for assisting a surgeon in navigating a medical instrument (306) equipped with a localizer, the navigation system (300) comprising
- a navigation unit according to at least one of claims 1 to 10,
- a localizer of a medical instrument (306), the localizer being configured for providing a localizer signal, the localizer being operatively connected to a first localizer input port of the navigation unit, and/or
- a patient localizer (314), the patient localizer (314) being configured for providing a patient localizer signal, the patient localizer (314) being operatively connected to a second localizer input port of the navigation unit, and
- a monitor (320) that is connected to the navigation unit and configured for displaying a position of the medical instrument (306).

12. A method for visually indicating a localizer operating state of a localizer, the method comprising the steps of
- operatively connecting a localizer to a navigation unit, the localizer being configured for capturing a navigation field and for providing a localizer signal,
- generating a navigation field for determining position and orientation of the localizer in the navigation field,
- receiving a localizer signal from the localizer and processing the received localizer signal in order to determine position and orientation of the localizer in the navigation field,
- determining a localizer operating state based on the received localizer signal and whether or not the received localizer signal represents position and orientation of the localizer in the navigation field, and
- controlling at least one operating state indicating light element that is assigned to a localizer input port used to connect the localizer to the navigation unit to indicate visually the determined localizer operating state of the localizer.

13. The method of claim 12, comprising the steps of
- determining an internal operating state of the navigation unit, and
- controlling at least one internal state indicating light element to indicate visually the determined internal operating state of the navigation unit.

14. A program means that is configured for causing a navigation unit according to one of claims 1 to 11 to perform the steps of
- generating a navigation field for determining position and orientation of the localizer in the navigation field,
- receiving a localizer signal from the localizer and processing the received localizer signal in order to determine position and orientation of the localizer in the navigation field,
- determining a localizer operating state based on whether or not the position detection unit has received a localizer signal of a localizer and whether or not the position detection unit has determined position and orientation of a localizer in a navigation field from a received localizer signal and optionally determining an internal operating state of the navigation unit, and
- outputting the determined localizer operating state of a localizer and optionally the determined internal operating state of the navigation unit, and
- controlling at least one operating state indicating light element that is assigned to a localizer input port used to connect the localizer to the navigation unit to indicate visually the determined localizer operating state of the localizer.

15. A persistent storage medium on which the program means according to claim 14 is stored.

## Patentansprüche

1. Eine Navigationseinheit, die dazu konfiguriert ist, einen Chirurgen bei der Navigation eines mit einem Lokalisator ausgestatteten medizinischen Instruments (306) zu unterstützen, wobei die Navigationseinheit ein Gehäuse (102, 202) mit Wänden, mindestens einen Lokalisator-Eingangsanschluss, eine Positionserfassungseinheit, eine Zustandsbestimmungseinheit (110), eine Steuereinheit (112) und mindestens ein Betriebszustandsanzeige-Leuchtelement umfasst, wobei
- der mindestens eine Lokalisator-Eingangsanschluss zum Empfangen eines Lokalisatorsignals konfiguriert ist, das von einem Lokalisator bereitgestellt wird, der mit dem Lokalisator-Eingangsanschluss wirkverbunden ist, und
- das mindestens eine Betriebszustandsanzeige-Lichtelement mit der Steuereinheit (112) verbunden und in eine der Gehäusewände (102, 202) integriert ist, so dass das vom Betriebszustandsanzeige-Lichtelement emittierte Licht von außerhalb des Gehäuses (102, 202) sichtbar ist,
wobei das Gehäuse (102, 202) mindestens
- die Positionserfassungseinheit, die mit dem Lokalisator-Eingangsanschluss verbunden ist, um ein Lokalisatorsignal zu empfangen, und die so konfiguriert ist, dass sie das empfangene Lokalisatorsignal verarbeitet, um die Position und Ausrichtung des Lokalisators zu bestimmen,
- die Zustandsbestimmungseinheit (110), die mit der Positionserfassungseinheit verbunden ist und so konfiguriert ist, dass sie mindestens einen Lokalisator-Betriebszustand eines angeschlossenen Lokalisators auf der Grundlage davon bestimmt, ob die Positionserfassungseinheit ein Lokalisatorsignal empfangen hat und ob die Positionserfassungseinheit die Position und Ausrichtung des Lokalisators aus einem empfangenen Lokalisatorsignal bestimmt hat, und
- die Steuereinheit (112), die mit der Zustandsbestimmungseinheit (110) verbunden ist und so konfiguriert ist, dass sie das mindestens eine Betriebszustandsanzeige-Leuchtelement mindestens auf der Grundlage des von der Zustandsbestimmungseinheit (110) ermittelten Lokalisator-Betriebszustands steuert,
beherbergt
wobei das mindestens eine Betriebszustandsanzeige-Lichtelement mindestens zum visuellen Anzeigen eines von mehreren Lokalisator-Betriebszuständen konfiguriert ist und von der Steuereinheit (112) mindestens zum visuellen Anzeigen des von der Zustandsbestimmungseinheit (110) ermittelten Lokalisator-Betriebszustands gesteuert werden kann.

2. Die Navigationseinheit nach Anspruch 1, wobei die Zustandsbestimmungseinheit (110) so konfiguriert ist, dass sie die Bestimmung auf einem Signal-Rausch-Verhältnis eines von dem angeschlossenen Lokalisators bereitgestellten Lokalisatorsignals basiert.

3. Die Navigationseinheit nach Anspruch 1 oder 2, wobei die Zustandsbestimmungseinheit (110) so konfiguriert ist, dass sie mehrere Schwellenwerte verwendet, die jeweils einem anderen Betriebszustand des Lokalisators zugeordnet sind, um den Betriebszustand eines angeschlossenen Lokalisators zu bestimmen.

4. Die Navigationseinheit nach mindestens einem der Ansprüche 1 bis 3, wobei die Zustandsbestimmungseinheit (110) so konfiguriert ist, dass sie ein Signalmuster eines von einem angeschlossenen Lokalisator bereitgestellten Lokalisatorsignals analysiert, um dessen Lokalisator -Betriebszustand zu bestimmen.

5. Die Navigationseinheit nach mindestens einem der Ansprüche 1 bis 4, wobei die Zustandsbestimmungseinheit (110) einen Speicher zum Speichern einer Anzahl von Betriebszuständen mindestens eines Lokalisators umfasst und wobei die Zustandsbestimmungseinheit (110) so konfiguriert ist, dass sie den Lokalisator-Betriebszustand eines angeschlossenen Lokalisators, dessen Lokalisator-Betriebszustände im Speicher gespeichert sind, bestimmt, indem sie ein von der Positionserfassungseinheit empfangenes Lokalisatorsignal einem der gespeicherten Lokalisator-Betriebszustände des angeschlossenen Lokalisators zuordnet.

6. Die Navigationseinheit nach mindestens einem der Ansprüche 1 bis 5, wobei die Zustandsbestimmungseinheit (110) so konfiguriert ist, dass sie bestimmt, ob ein Lokalisator mit der Navigationseinheit verbunden ist und/oder ein verbundener Lokalisator nicht oder nicht vollständig funktioniert und/oder sich ein verbundener Lokalisator in einem Arbeitsbereich befindet, in dem ein Signal-Rausch-Verhältnis eines vom Lokalisator des medizinischen Instruments (306) bereitgestellten Lokalisatorsignals über einem vordefinierten Schwellenwert liegt.

7. Die Navigationseinheit nach mindestens einem der Ansprüche 1 bis 6, wobei die Zustandsbestimmungseinheit (110) so konfiguriert ist, dass sie bestimmt, ob ein als Patientenlokalisator (314) verwendeter Lokalisator mit der Navigationseinheit verbunden ist und/oder ein verbundener Patientenlokalisator (314) nicht oder nicht vollständig funktioniert und/oder ein verbundener Patientenlokalisator (314) eine Verzerrung oder Störung des Navigationsfeldes erkennt und/oder sich ein angeschlossener Patientenlokalisator (314) in einem Arbeitsbereich befindet, in dem ein Signal-Rausch-Verhältnis eines vom Patientenlokalisator (314) bereitgestellten Patientenlokalisatorsignals über einem vordefinierten Schwellenwert liegt.

8. Die Navigationseinheit nach mindestens einem der Ansprüche 1 bis 7, wobei das mindestens eine Betriebszustandsanzeige-Leuchtelement in verschiedenen Betriebsmodi betrieben werden kann, wobei jeder Betriebsmodus einem anderen Lokalisator-Betriebszustand eines angeschlossenen Lokalisators zugeordnet ist, wobei das mindestens eine Betriebszustandsanzeige-Leuchtelement von der Steuereinheit (112) so gesteuert wird, dass es in dem Betriebsmodus betrieben wird, der dem ermittelten Lokalisator-Betriebszustand zugeordnet ist.

9. Die Navigationseinheit nach mindestens einem der Ansprüche 1 bis 8, die ferner mindestens ein internes Betriebszustandsanzeigeelement umfasst, wobei
- die Zustandsbestimmungseinheit (110) zum Bestimmen eines internen Betriebszustands der Navigationseinheit konfiguriert ist,
- die Steuereinheit (112) so konfiguriert ist, dass sie das mindestens eine interne Zustandsanzeigelichtelement auf der Grundlage des von der Zustandsbestimmungseinheit (110) bestimmten internen Betriebszustands der Navigationseinheit steuert,
wobei das mindestens eine interne Zustandsanzeige-Lichtelement so konfiguriert ist, dass es einen von mehreren internen Betriebszuständen der Navigationseinheit visuell anzeigt, und von der Steuereinheit (112) so gesteuert werden kann, dass es den von der Zustandsbestimmungseinheit (110) ermittelten internen Betriebszustand visuell anzeigt.

10. Die Navigationseinheit nach Anspruch 9, wobei die Zustandsbestimmungseinheit (110) so konfiguriert ist, dass sie bestimmt, ob die Navigationseinheit hochfährt und/oder sich in einem Berechnungszustand befindet und/oder sich in einem Navigationsmodus befindet und/oder eine Benutzerinteraktion erfordert und/oder ob die Bestimmung der Position und Ausrichtung eines mit der Navigationseinheit verbundenen Lokalisators ungenau ist oder unterbrochen wird und/oder ob ein mit der Navigationseinheit verbundener Lokalisator einen Arbeitsbereich verlässt und/oder ob ein Fehler erkannt wird.

11. Navigationssystem (300) zur Unterstützung eines Chirurgen bei der Navigation eines mit einem Lokalisator ausgestatteten medizinischen Instruments (306), wobei das Navigationssystem (300) umfasst
- eine Navigationseinheit gemäß mindestens einem der Ansprüche 1 bis 10,
- einen Lokalisator eines medizinischen Instruments (306), wobei der Lokalisator so konfiguriert ist, dass er ein Lokalisatorsignal liefert, und wobei der Lokalisator funktionsfähig mit einem ersten Lokalisator-Eingangsanschluss der Navigationseinheit verbunden ist, und/oder
- einen Patientenlokalisator (314), wobei der Patientenlokalisator (314) so konfiguriert ist, dass er ein Patientenlokalisatorsignal bereitstellt, wobei der Patientenlokalisator (314) funktionsfähig mit einem zweiten Lokalisator-Eingangsanschluss der Navigationseinheit verbunden ist, und
- einen Monitor (320), der mit der Navigationseinheit verbunden und zum Anzeigen einer Position des medizinischen Instruments (306) konfiguriert ist.

12. Verfahren zum visuellen Anzeigen eines Lokalisator-Betriebszustands eines Lokalisators, wobei das Verfahren die folgenden Schritte umfasst
- Betriebsmäßiges Verbinden eines Lokalisators mit einer Navigationseinheit, wobei der Lokalisator so konfiguriert ist, dass es ein Navigationsfeld erfasst und ein Lokalisatorsignal bereitstellt,
- Erzeugen eines Navigationsfeldes zum Bestimmen der Position und Ausrichtung des Lokalisators im Navigationsfeld,
- Empfangen eines Lokalisatorsignals vom Lokalisator und Verarbeiten des empfangenen Lokalisatorsignals, um die Position und Ausrichtung des Lokalisators im Navigationsfeld zu bestimmen,
- Bestimmen eines Lokalisator-Betriebszustands auf der Grundlage des empfangenen Lokalisatorsignals und der Frage, ob das empfangene Lokalisatorsignal die Position und Ausrichtung des Lokalisators im Navigationsfeld darstellt oder nicht, und
- Steuern mindestens eines Betriebszustandsanzeigeelements, das einem Lokalisator-Eingangsanschluss zugeordnet ist, der zum Verbinden des Lokalisators mit der Navigationseinheit verwendet wird, um den ermittelten Betriebszustand des Lokalisators visuell anzuzeigen.

13. Verfahren nach Anspruch 12, umfassend die folgenden Schritte
- Bestimmen eines internen Betriebszustands der Navigationseinheit und
- Steuern mindestens eines internen Zustandsanzeigeelements, um den ermittelten internen Betriebszustand der Navigationseinheit visuell anzuzeigen.

14. Ein Programm, das so konfiguriert ist, dass es eine Navigationseinheit gemäß einem der Ansprüche 1 bis 11 veranlasst, die folgenden Schritte auszuführen
- Erzeugen eines Navigationsfeldes zum Bestimmen der Position und Ausrichtung des Lokalisators im Navigationsfeld,
- Empfangen eines Lokalisatorsignals vom Lokalisator und Verarbeiten des empfangenen Lokalisatorsignals, um die Position und Ausrichtung des Lokalisators im Navigationsfeld zu bestimmen,
- Bestimmen eines Lokalisator-Betriebszustands basierend darauf, ob die Positionserfassungseinheit ein Lokalisator-Signal eines Lokalisators empfangen hat und ob die Positionserfassungseinheit die Position und Ausrichtung eines Lokalisators in einem Navigationsfeld aus einem empfangenen Lokalisatorsignal bestimmt hat, und optional Bestimmen eines internen Betriebszustands der Navigationseinheit, und
- Ausgeben des ermittelten Betriebszustands eines Lokalisators und optional des ermittelten internen Betriebszustands der Navigationseinheit, und
- Steuern mindestens eines Betriebszustandsanzeige-Leuchtelements, das einem Lokalisator-Eingangsanschluss zugeordnet ist, der zum Verbinden des Lokalisators mit der Navigationseinheit verwendet wird, um den ermittelten Betriebszustand des Lokalisators visuell anzuzeigen.

15. Ein dauerhaftes Speichermedium, auf dem das Programm gemäß Anspruch 14 gespeichert ist.

## Revendications

1. Une unité de navigation, qui est configurée pour aider un chirurgien à naviguer un instrument (306) médical muni d'un dispositif de localisation, l'unité de navigation comportant un boîtier (102, 202) comportant des parois, au moins un port d'entrée de dispositif de localisation, une unité de détection de position, une unité (110) de détermination d'état, une unité (112) de commande et au moins un élément lumineux indiquant un état de fonctionnement, dans laquelle
- le au moins un port d'entrée de dispositif de localisation étant configuré pour recevoir un signal de dispositif de localisation fourni par un dispositif de localisation connecté de manière fonctionnelle au port d'entrée de dispositif de localisation, et
- le au moins un élément lumineux indiquant un état de fonctionnement étant connecté à l'unité (112) de commande et étant intégré dans l'une des parois du boîtier (102, 202), de sorte que de la lumière émise de l'élément lumineux indiquant un état de fonctionnement est visible de l'extérieur du boîtier (102, 202),
le boîtier (102, 202) recevant au moins en son sein
- l'unité de détection de position, qui est connectée au port d'entrée de dispositif de localisation pour recevoir un signal de dispositif de localisation et configurée pour traiter le signal de dispositif de localisation reçu afin de déterminer la position et l'orientation du dispositif de localisation,
- l'unité (110) de détermination d'état, qui est connectée à l'unité de détection de position et est configurée pour déterminer au moins un état de fonctionnement du dispositif de localisation d'un dispositif de localisation connecté sur la base du point de savoir si oui ou non l'unité de détection de position a reçu un signal de dispositif de localisation et du point de savoir si oui ou non l'unité de détection de position a déterminé la position et l'orientation du dispositif de localisation à partir d'un signal de dispositif de localisation reçu, et
- l'unité (112) de commande, qui est connectée à l'unité (110) de détermination d'état et configurée pour commander le au moins un élément lumineux indiquant l'état de fonctionnement au moins sur la base de l'état de fonctionnement du dispositif de location déterminé par l'unité (110) de détermination d'état,
dans laquelle le au moins un élément lumineux indiquant un état de fonctionnement est configuré au moins pour indiquer visuellement l'un d'une pluralité d'états de fonctionnement de dispositif de localisation et peut être commandé par l'unité (112) de commande au moins pour visuellement indiquer celui des états de fonctionnement de dispositif de localisation qui a été déterminé par l'unité (110) de détermination d'état.

2. L'unité de navigation de la revendication 1, dans laquelle l'unité (110) de détermination d'état est configurée pour baser la détermination sur un rapport signal à bruit d'un signal de dispositif de localisation fourni par le dispositif de localisation connecté.

3. L'unité de navigation de la revendication 1 ou 2, dans laquelle l'unité (110) de détermination d'état est configurée pour utiliser une pluralité de valeurs seuil, chacune affectée à un état de fonctionnement de dispositif de localisation différent pour déterminer l'état de fonctionnement du dispositif de localisation d'un dispositif de localisation connecté.

4. L'unité de navigation d'au moins l'une des revendications 1 à 3, dans laquelle l'unité (110) de détermination d'état est configurée pour analyser un motif de signal d'un signal de dispositif de localisation fourni par un dispositif de localisation connecté pour déterminer son état de fonctionnement de dispositif de localisation.

5. L'unité de navigation d'au moins l'une des revendications 1 à 4, dans laquelle l'unité (110) de détermination d'état comporte une mémoire pour mémoriser un nombre d'états de fonctionnement d'au moins un dispositif de localisation et dans laquelle l'unité (110) de détermination d'état est configurée pour déterminer l'état de fonctionnement du dispositif de localisation d'un dispositif de localisation connecté dont les états de fonctionnement de dispositif de localisation sont mémorisés dans la mémoire en affectant un signal de dispositif de localisation reçu par l'unité de détection de position à l'un des états de fonctionnement de dispositif de localisation mémorisé du dispositif de localisation connecté.

6. L'unité de navigation d'au moins l'une des revendications 1 à 5, dans laquelle l'unité (110) de détermination d'état est configurée pour déterminer si un dispositif de localisation est connecté à l'unité de navigation, et/ou si un dispositif de localisation connecté ne fonctionne pas ou ne fonctionne pas dans toute sa mesure, et/ou si un dispositif de localisation connecté se trouve dans un espace de travail dans lequel un rapport signal à bruit d'un signal de dispositif de localisation fourni par le dispositif de localisation de l'instrument (306) médical se trouve au-dessus d'un seuil défini à l'avance.

7. L'unité de navigation d'au moins l'une des revendications 1 à 6, dans laquelle l'unité (110) de détermination d'état est configurée pour déterminer si un dispositif de localisation utilisé en tant qu'un dispositif (314) de localisation de patient est connecté à l'unité de navigation, et/ou si un dispositif (314) de localisation de patient connecté ne fonctionne pas ou ne fonctionne pas dans toute sa mesure, et/ou si un dispositif (314) de localisation de patient connecté détecte une distorsion ou une interférence du champ de navigation, et/ou si un dispositif (314) de localisation de patient connecté se trouve à l'intérieur d'un espace de travail dans lequel un rapport signal à bruit d'un signal de dispositif de localisation de patient fourni par le dispositif (314) de localisation de patient est au-dessus d'un seuil défini à l'avance.

8. L'unité de navigation d'au moins l'une des revendications 1 à 7, dans laquelle le au moins un élément lumineux indiquant un état de fonctionnement peut être mis en fonctionnement dans divers modes de fonctionnement, chaque mode de fonctionnement étant affecté à un état de fonctionnement d'un dispositif de localisation différent d'un dispositif de localisation connecté, dans laquelle le au moins un élément lumineux indiquant un état de fonctionnement est commandé par l'unité (112) de commande pour fonctionner dans le mode de fonctionnement qui est affecté à l'état de fonctionnement du dispositif de localisation déterminé.

9. L'unité de navigation d'au moins l'une des revendications 1 à 8, comportant en outre au moins un élément lumineux indiquant un état interne, dans lequel
- l'unité (110) de détermination d'état est configurée pour déterminer un état de fonctionnement interne de l'unité de navigation,
- l'unité (112) de commande est configurée pour commander le au moins un élément lumineux indiquant un état interne sur la base de l'état de fonctionnement interne de l'unité de navigation déterminée par l'unité (110) de détermination d'état,
dans laquelle le au moins un élément lumineux indiquant un état interne est configuré pour indiquer visuellement l'un d'une pluralité d'états de fonctionnent internes de l'unité de navigation et peut être commandé par l'unité (112) de commande pour visuellement indiquer celui des états de fonctionnement internes qui a été déterminé par l'unité (110) de détermination d'état.

10. L'unité de navigation de la revendication 9, dans laquelle l'unité (110) de détermination d'état est configurée pour déterminer si l'unité de navigation redémarre, et/ou est dans un état de calcul, et/ou est dans un état de navigation, et/ou nécessite une interaction de l'utilisateur, et/ou si la détermination de position et d'orientation d'un dispositif de localisation connecté à l'unité de navigation est imprécise ou interrompue, et/ou si un dispositif de localisation connecté à l'unité de navigation quitte un espace de travail, et/ou si une erreur est détectée.

11. Un système (300) de navigation pour aider un chirurgien à naviguer un instrument médical (306) équipé d'un dispositif de localisation, le système (300) de navigation comportant
- une unité de navigation suivant au moins l'une des revendications 1 à 10,
- un dispositif de localisation d'un instrument (306) médical, le dispositif de localisation étant configuré pour fournir un signal de dispositif de localisation, le dispositif de localisation étant connecté de manière fonctionnelle à un premier port d'entrée de dispositif de localisation de l'unité de navigation, et/ou
- un dispositif (314) de localisation de patient, le dispositif (314) de localisation de patient étant configuré pour fournir un signal de dispositif de localisation de patient, le dispositif (314) de localisation de patient étant connecté de manière fonctionnelle à un deuxième port d'entrée de dispositif de localisation d'unité de navigation, et
- un dispositif (320) d'affichage, qui est connecté à l'unité de navigation et qui est configuré pour afficher une position de l'instrument (306) médical.

12. Un procédé pour indiquer visuellement un état de fonctionnement de dispositif de localisation d'un dispositif de localisation, le procédé comportant les étapes dans lesquelles :
- on connecte de manière fonctionnelle un dispositif de localisation à une unité de navigation, le dispositif de localisation étant configuré pour capturer un champ de navigation et pour fournir un signal de dispositif de localisation,
- on produit un champ de navigation pour déterminer la position et l'orientation du dispositif de localisation dans le champ de navigation,
- on reçoit un signal de dispositif de localisation du dispositif de localisation et on traite le signal de dispositif de localisation reçu afin de déterminer la position et l'orientation du dispositif de localisation dans le champ de navigation,
- on détermine un état de fonctionnement de dispositif de localisation sur la base du signal de dispositif de localisation reçu et du point de savoir si oui ou non le signal de dispositif de localisation reçu représente la position et l'orientation du dispositif de localisation dans le champ de navigation, et
- on commande au moins un élément lumineux indiquant un état de fonctionnement, qui est affecté à un port d'entrée de dispositif de localisation utilisé pour connecter le dispositif de localisation à l'unité de navigation pour indiquer visuellement l'état de fonctionnement de dispositif de localisation déterminé du dispositif de localisation.

13. Le procédé de la revendication 12, comportant les étapes dans lesquelles :
- on détermine un état de fonctionnement interne de l'unité de navigation, et
- on commande au moins un élément lumineux indiquant un état interne pour indiquer visuellement l'état de fonctionnement interne déterminé de l'unité de navigation.

14. Des moyens de programmation, qui sont configurés pour faire en sorte que l'unité de navigation suivant l'une des revendications 1 à 11 effectue les étapes dans lesquelles :
- on produit un champ de navigation pour déterminer la position et l'orientation du dispositif de localisation dans le champ de navigation,
- on reçoit un signal de dispositif de localisation du dispositif de localisation et on traite le signal de dispositif de localisation reçu afin de déterminer la position et l'orientation du dispositif de localisation dans le champ de navigation,
- on détermine un état de fonctionnement de dispositif de localisation fondé sur le point de savoir si oui ou non l'unité de détection de position a reçu un signal de dispositif de localisation d'un dispositif de localisation et du point de savoir si oui ou non l'unité de détection de position a déterminé la position et l'orientation d'un dispositif de localisation dans un champ de navigation à partir d'un signal de dispositif de localisation reçu et éventuellement on détermine un état de fonctionnement interne de l'unité de navigation, et
- on émet en sortie l'état de fonctionnement du dispositif de localisation déterminé d'un dispositif de localisation et éventuellement l'état de fonctionnement interne déterminé de l'unité de navigation, et
- on commande au moins un élément lumineux indiquant un état de fonctionnement qui est affecté à un port d'entrée du dispositif de localisation utilisé pour connecter le dispositif de localisation à l'unité de navigation pour indiquer visuellement l'état de fonctionnement du dispositif de localisation déterminé du dispositif de localisation.

15. Un support de stockage persistant dans lequel les moyens de programmation suivant la revendication 14 sont mémorisés.
